(19) Europäisches Patentamt
European Patent Office
Office européen des brevets

(11) Publication number: **0 088 522**
**A2**

# (12) EUROPEAN PATENT APPLICATION

(21) Application number: **83300647.1**

(22) Date of filing: **09.02.83**

(51) Int. Cl.³: **C 07 C 107/02,** C 08 F 4/04

(30) Priority: **04.03.82 GB 8206315**
**31.03.82 GB 8209387**

(43) Date of publication of application: **14.09.83**
**Bulletin 83/37**

(84) Designated Contracting States: **BE DE FR GB IT NL**

(71) Applicant: **IMPERIAL CHEMICAL INDUSTRIES PLC, Imperial Chemical House Millbank, London SW1P 3JF (GB)**

(72) Inventor: **Davies, Stephen Parry, 26 Clara Street South Yarra, Melbourne Victoria 3141 (AU)**
Inventor: **Thompson, Morice William, "Urishay" Highfield Lane Cox Green, Maidenhead Berkshire (GB)**

(74) Representative: **Wood, Dennis John Cecil et al, Imperial Chemical Industries PLC Legal Department: Patents Thames House North Millbank, London SW1P 4QG (GB)**

## (54) Novel esters or amides of azobiscarboxylic acids and their use as polymerisation initiators.

(57) Novel azo compounds are esters or substitued amides of an azobiscarboxylic acid defined by the formula

$$\left[ =N-\underset{\displaystyle R_2}{\overset{\displaystyle CN}{C}}.\ CH_2CH_2CO.\ X(C_nH_{2n}O)_p\ CH_2\ \overset{\displaystyle R_1}{C}H.OR \right]_2$$

in which R and $R_1$ are independently hydrogen or a methyl group, $R_2$ is an alkyl group containing from 1 to 4 carbon atoms, X is oxygen or a group $NR_3$ where $R_3$ is hydrogen or a grouping $-(C_nH_{2n}O)_p\ CH_2\overset{R_1}{C}H.OR$, n has the value 2, 3 or 4 and p is zero or an integer from 1 to 100, preferably from 1 to 50.

The compounds are useful as free radical-forming initiators for the polymerisation of ethylenically unsaturated monomers, in either organic or aqueous media according to the solubility properties of the compounds.

EP 0 088 522 A2

82210/EP

0088522

# NOVEL ESTERS OR AMIDES OF AZOBISCARBOXYLIC ACIDS AND THEIR USE AS POLYMERISATION INITIATORS

This invention relates to novel organic azo compounds which are useful as initiators for the polymerisation of ethylenically unsaturated monomers, more particularly for such polymerisation when carried out in aqueous or non-aqueous media.

The use of organic azo compounds as initiators in the aqueous emulsion polymerisation of ethylenically unsaturated monomers is well known and extensively described in the literature. Illustrative of such compounds are 2,2'-azobis(2-methylpropionitrile), 4,4'-azobis(4-cyanopentanoic acid), 2,2'-azobis(2-cyanoethyl-p-benzoic acid), 4,4'-azobis(4-cyano-n-pentanol), 2,2'-azobis(4-amino-2,4-dimethylvaleronitrile), and 1,1'-azobis(1-cyanocyclohexane).

According to the present invention there are provided azo compounds having the general formula

$$\left[=N-\underset{R_2}{\overset{CN}{C}}.CH_2CH_2CO.X.(C_nH_{2n}O)_p\ CH_2\overset{R_1}{CH}.\ OR\right]_2 \qquad (I)$$

in which R and $R_1$ are independently hydrogen or a methyl group, $R_2$ is an alkyl group containing from 1 to 4 carbon atoms, X is oxygen or a group $NR_3$ where $R_3$ is hydrogen or a grouping $-(C_nH_{2n}O)_p\ CH_2\overset{R_1}{CH}.OR$, n has the value 2, 3 or 4 and p is zero or an integer from 1 to 100, preferably from 1 to 50.

The compounds of the invention are thus either esters or substituted amides of an azobiscarboxylic acid

and they may contain hydroxyl groups and/or oxyalkylene groups. The preferred compounds are those derived from 4,4'-azobis(cyanopentanoic acid) (where $R_2$ in formula (I) is a methyl group).

As an illustration of the ester group of compounds, where X in formula (I) is oxygen, there may be mentioned the ester of 4,4'-azobis(cyanopentanoic acid) with the monomethyl ether of polyethylene glycol of molecular weight 2000; here R is methyl, $R_1$ is hydrogen, $R_2$ is methyl, n is 2 and p is approximately 44.

As illustrations of the amide group of compounds, where X in formula (I) is $NR_3$, there may be mentioned 4,4'-azobis(cyanopentanoic acid) ethanolamide, where R, $R_1$ and $R_3$ are hydrogen, $R_2$ is methyl, n is 2 and p is zero; 4,4'-azobis(cyanopentanoic acid)diethanolamide, where R and $R_1$ are hydrogen, $R_2$ is methyl, $R_3$ is $-CH_2CH_2OH$, n is 2 and p is zero; and the amide of 4,4'-azobis(cyanopentanoic acid) with α-amino-ω-hydroxypoly(oxyethylene) of molecular weight 1600, where R, $R_1$ and $R_3$ are hydrogen, $R_2$ is methyl, n is 2 and p is 33.

The compounds of the invention may readily be obtained by the reaction of the appropriate azobiscarboxylic acid halide with the appropriate hydroxy or amino compound, in known manner in the presence of a hydrogen halide acceptor. Thus, 4,4'-azobis(cyanopentanoyl chloride) can be obtained by treatment of the acid with thionyl chloride and then reacted with diethanolamine in the presence of pyridine or 2,6-lutidine. Where it is desired to produce an ester, i.e. a compound according to formula (I) where X is oxygen, it is preferred that the group R should be a methyl group; in other words, one hydroxyl group of the glycol or poly(oxyalkylene)glycol with which the acid halide

is reacted should be etherified, in order to avoid the formation of a complex condensation product. Suitable starting materials include ethylene glycol monomethyl ether, diethylene glycol monoethyl ether and the monomethyl ether of polyethylene glycol, mol.wt. 2000. However, in the case of the production of an amide, where X is $NR_3$ and the acid halide is reacted with an alkanolamine or a hydroxypoly-(oxyalkylene)amine, it is not necessary for the hydroxyl group to be etherified since the amino group is relatively much more reactive towards the carboxyl halide group. Suitable hydroxyamines include, for example, ethanolamine, diethanolamine, isopropanolamine, diisopropanolamine and α-amino-ω-hydroxypoly(oxyethylene) having a molecular weight of about 1600. The last-named of these substances can be obtained by oxyethylation of the ketimine derived from methyl isobutyl ketone and ethanolamine, followed by hydrolysis of the ketimine grouping.

During the reaction of the azocarboxylic acid halide with the hydroxy or amino compound, the temperature should be kept below 30°C and preferably below 10°C. The reaction may if desired be carried out in a suitable inert solvent, such as tetrahydrofuran.

Azo compounds according to the invention are valuable as free radical-forming initiators for the polymerisation of ethylenically unsaturated monomers. According to their physical properties, they may be especially suitable for specific polymerisation procedures. Thus, certain members of the class are preferentially soluble in organic media, for example those in which the oxyalkylene groups are mainly derived from propylene oxide; these are consequently suitable for initiating polymerisation of monomers in bulk or in solution. Other members of the class

are, in contrast, preferentially soluble in water, more particularly those in which the oxyalkylene groups are exclusively oxyethylene groups. These compounds are useful as initiators in aqueous emulsion polymerisation processes, or in the aqueous dispersion polymerisation processes described in published British Application No. 2 039 497A.

The invention is illustrated but not limited by the following Examples, in which parts are by weight.

## EXAMPLE 1

4,4'-Azobis(cyanopentanoic acid) (50g) was dissolved in tetrahydrofuran (500 ml) and pyridine (2 ml) was added. The mixture was stirred and cooled (ice/water) in a 1-litre Florentine flask fitted with a dropping funnel and calcium chloride drying tubes. Thionyl chloride (200 ml) was added dropwise with continued stirring and cooling over a period of 2 hours. After the mixture had then stood for 1 hour at 15°C, infra-red spectral examination showed complete conversion of the acid to the acid chloride. Excess thionyl chloride and solvent were then removed under reduced pressure, giving a viscous yellow liquid which was dissolved in dichloromethane (200 ml). The resulting solution was divided into two equal portions.

To one of the portions of acid chloride solution, there was added with stirring and cooling, over a period of 30 minutes, a solution of dry diisopropanolamine (65g, three times the stoichiometric amount) in dichloromethane. After standing at 20-25°C for 16 hours, the reaction mixture was filtered and the solvent removed under reduced pressure, giving a yellow-brown oil (47.6g). The oil was dissolved in methanol and passed through a column of "Amberlite" IR 120 ion-exchange resin ($H^+$ form) ("Amberlite" is a Registered

Trade Mark) which had previously been equilibrated with methanol, in order to remove any material bearing unreacted amino groups. Removal of solvent under reduced pressure gave a pale yellow liquid (30 g) which, from the results of infra-red spectral examination, was concluded to be essentially the diisopropanolamide of 4,4'-azobis(cyano-pentanoic acid) having the formula:-

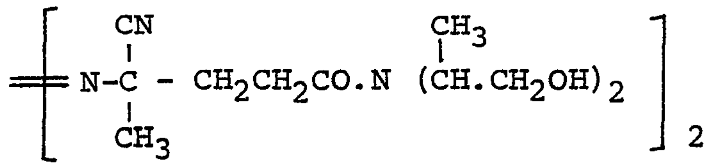

This product was insoluble in methyl methacrylate but soluble in water.

EXAMPLE 2

To the second of the portions of acid chloride solution obtained as described in Example 1 there was added with stirring and cooling over a period of 30 minutes, a solution of dry ethanolamine (30g, about three times the stoichiometric amount) in dichloromethane. After standing at 20-25°C for 16 hours, a brown precipitate had formed; this was removed by filtration and washed with dichloromethane (200 ml). The filtrate and washings were combined and the solvent removed under reduced pressure, giving a yellow oil (19g), which was dissolved in methanol and passed through a column of "Amberlite" IR 120 ion-exchange resin ($H^+$ form) which had previously been washed with methanol. Removal of the solvent under reduced pressure gave a yellow oil (9.8g) which, from the results of infra-red spectral examination, was concluded to be essentially the ethanolamide of 4,4'-azobis(cyanopentanoic acid) having the formula:-

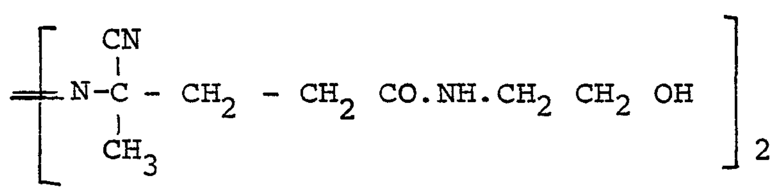

The product was readily soluble in water but much less soluble in methyl methacrylate. The infra-red spectrum exhibited bands <u>inter alia</u> at 1555 $cm^{-1}$, 1655 $cm^{-1}$, 1730 $cm^{-1}$, 2950 $cm^{-1}$ and 3300-3500 $cm^{-1}$.

## <u>EXAMPLE 3</u>

A solution in tetrahydrofuran (200g) of the acid chloride of 4,4'-azobis(cyanopentanoic acid), prepared by the method described in Example 1 from the acid (18g), was added dropwise, with stirring and cooling so as to keep the temperature below -10°C, to a solution of diethanolamine (90g, dried by azeotropic distillation with cyclohexane) in tetrahydrofuran (300g). The reaction mixture was left overnight, during which time it separated into two layers. The clear upper layer was decanted and concentrated under reduced pressure to give a pale yellow, viscous liquid which was soluble in water and insoluble in methyl methacrylate, On examination by infra-red spectroscopy it exhibited absorptions at 3325 $cm^{-1}$ and 2900 $cm^{-1}$ (hydroxyl groups), 2290 $cm^{-1}$ (nitrile), 1715 $cm^{-1}$ (ester) and 1610 $cm^{-1}$ (amide). The product was concluded to be essentially the diethanolamide of 4,4'-azobis(cyanopentanoic acid) having the formula

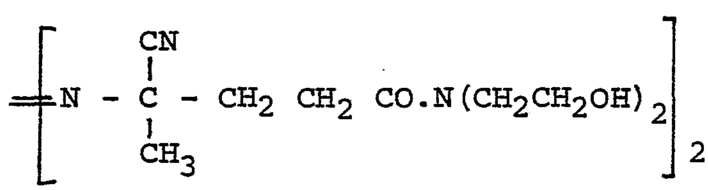

## <u>EXAMPLE 4</u>

A. <u>Preparation of α-Amino-ω-Hydroxypoly(oxyethylene)</u>

The ketimine from methylisobutylketone and ethanolamine, prepared by condensation of the starting materials in the presence of acid, was reacted with sufficient ethylene oxide to give a final molecular weight of

about 1600. In order to hydrolyse the ketimine grouping, 100g of the oxyethylated material was dissolved in water, acetic anhydride (30g) was added and the mixture was allowed to stand at room temperature for 16 hours. The water and other volatile material were removed under reduced pressure and the residue was dried by azeotropic distillation with toluene (50g). This product was believed to contain some oxyethylated ethanolamine, arising from the presence of some unchanged ethanolamine in the ketimine, and this impurity was removed by the following procedure. The dried product was dissolved in acetic anhydride (200 ml), heated at reflux temperature for 90 minutes and then kept in a closed vessel at room temperature for 16 hours. Volatile material was removed under reduced pressure and the residue was dissolved in chloroform (about 150 ml), then washed with aqueous sodium carbonate solution. The organic layer was evaporated under reduced pressure and the residue suspended in water (250 ml); the suspension was filtered through beds of powdered diatomaceous earth ("Celite" 545 : Registered Trade Mark) and of activated charcoal and the filtrate passed successively through columns of anionic and cationic ion exchange resins ("Amberlite" IRA 400 ($OH^-$ form) and "Amberlite" IR 120 ($H^+$ form) respectively : Registered Trade Marks). The volume of eluate was brought to about 200 ml with water and sodium hydroxide (10g) was added. The mixture was heated with stirring under reflux for 6 hours, neutralised with 2N-sulphuric acid and extracted with chloroform (100 ml). The extract was washed with aqueous sodium carbonate solution (3 x 50 ml) and then with portions of distilled water until the chloroform layer was neutral. The solvent was removed under reduced pressure and the residue dried by heating at 60°C under 1mm Hg pressure. The product was essentially pure α-amino-ω-hydroxypoly(oxyethylene) of mol.wt. 1600.

B. Reaction with 4,4'-azobis(cyanopentanoyl chloride).

4,4'-azobis(cyanopentanoic acid) (7.0g) was dissolved in tetrahydrofuran (60 ml) and pyridine (7 drops) added. Thionyl chloride (25 ml) was then added dropwise with stirring and cooling, keeping the temperature below 20°C, and the mixture allowed to stand at room temperature overnight. Volatile reagents and the solvent were removed under vacuum (oil-pump) and the residual white slurry was mixed with tetrahydrofuran (30 ml) and a portion (2 ml) of the mixture was added with stirring to a solution of the product of stage A (10 g) in tetrahydrofuran (25 ml) containing pyridine (15 drops). The resulting mixture was stirred at room temperature for 16 hours and solid material then removed by filtration. Examination of the filtered solution by infra-red spectroscopy showed, in addition to peaks attributable to polyethyleneglycol groupings and tetrahydrofuran, small absorptions at 1645 $cm^{-1}$ (amide grouping) and at 1735 $cm^{-1}$ (ester grouping), the former peak having about twice the area of the latter. This indicated that a minor part of the aminohydroxypoly(oxyethylene) had reacted with the acid chloride via the hydroxyl group rather than via the amino group. The solution was evaporated under reduced pressure, giving a colourless, waxy solid (14.5g). The greater part (13.5g) of this material was freed from the ester impurity by dissolving it in methanol and passing it through a column of "Amberlite" IR 120 ($H^+$ form) ion-exchange resin, following which the methanol was removed under reduced pressure to yield a colourless waxy solid (9.0g) which was soluble in both water and methyl methacrylate separately but preferentially soluble in water in the presence of methyl methacrylate.

The product was concluded to be essentially the

 

amide of 4,4'-azobis(cyanopentanoic acid) with α-amino-ω-hydroxypoly(oxyethylene), having the formula:-

$$\left[ =N-\overset{\displaystyle CN}{\underset{\displaystyle CH_3}{\overset{|}{\underset{|}{C}}}}-CH_2\;CH_2.\;CO\;NH(C_2H_4O)_{33}\;CH_2\;CH_2\;OH \right]_2$$

## EXAMPLE 5

4,4'-azobis(cyanopentanoic acid) (18g) and pyridine (1 ml) were dissolved in tetrahydrofuran (200 ml), and thionyl chloride (70 ml) was then added dropwise to the stirred, cooled solution over a period of 2 hours. After a further 1 hour, excess thionyl chloride and solvent were removed under reduced pressure and the residue was dissolved in tetrahydrofuran (200 ml). A solution of the monomethyl ether of polyethylene glycol, mol.wt. 200 (100g) in tetrahydrofuran (450 ml) was added dropwise to this solution, over a period of 12 hours, and the reaction mixture was kept at $-15^{o}C$ for 2 days. The reaction mixture was then concentrated under reduced pressure, dissolved in methanol and the solution passed through a column of "Amberlite" IRA 400 ($OH^-$ form) ion-exchange resin. The eluate was concentrated under reduced pressure, giving a residue (110g) which on examination by infra-red spectroscopy showed a small peak at 2250 $cm^{-1}$ (nitrile group) and a large peak at 1740 $cm^{-1}$ (ester group). This material was a waxy solid which was soluble in both water and methyl methacrylate separately but preferentially soluble in water in the presence of methyl methacrylate. The product was concluded to be essentially the ester of 4,4'-azobis(cyanopentanoic acid) with the monomethyl ether of polyethylene glycol, mol.wt. 2000, having the formula:-

$$\left[ =N-\overset{\displaystyle CN}{\underset{\displaystyle CH_3}{\overset{|}{\underset{|}{C}}}}-CH_2\;CH_2\;CO.O(C_2H_4O)_{44}\;CH_2\;CH_2\;OCH_3 \right]_2$$

The following Examples 6 - 8 illustrate the use of some of the above-described initiators in an aqueous dispersion polymerisation process as described in published British Application No. 2 039 497A. The polymer latices obtained were subjected to determination of particle size and distribution by means of the "Nanosizer" (Registered Trade Mark), an instrument marketed in the U.K. by Coulter Electronics Ltd. The results of this determination are expressed by two numbers, e.g. 3:361; the first number is a measure of the degree of polydispersity of the latex particles on an arbitrary scale from 0 (monodisperse) to 10, and the second number is the average size of the particles in nanometres.

EXAMPLE 6

The amide of 4,4'-azobis(cyanopentanoic acid) with α-amino-ω-hydroxypoly(oxyethylene) of mol.wt. 1600 (1.0g) was dissolved under an atmosphere of nitrogen, in a mixture of water (30g) and methanol (50g) contained in a roundbottomed flask and stirred by means of a magnetic follower. The flask was fitted with a reflux condenser. Methyl methacrylate (3g) and butyl acrylate (3g) were added and the contents of the flask were then heated to 65 - 70°C by means of a water-bath. After 25 minutes, a milky-white turbidity appeared and, after a total of three hours, a bit-free polymer latex was obtained. "Nanosizer" examination of the product gave a result of 1:613.

EXAMPLE 7

The procedure of Example 6 was repeated, using a monomer charge of methyl methacrylate (6g) in place of the mixture of methyl methacrylate and butyl acrylate.

After the reaction mixture had been heated for 1½ hours, examination of the resulting latex by "Nanosizer" gave the result 6:612.

EXAMPLE 8

The methacrylic acid ester of the monomethyl ether of polyethylene glycol, mol.wt. 2000 (1.38g) was dissolved in a mixture of water (30g) and methanol (50g); methyl methacrylate (6g) and the diethanol amide of 4,4'-azobis(cyanopentanoic acid)(0.138g) were then added. The reaction mixture was stirred and heated at 65 - 70°C (water bath) for 3 hours. The resulting latex was free from bits and "Nanosizer" determination gave a result of 3:361.

WE CLAIM :-

1. Azo compounds having the general formula

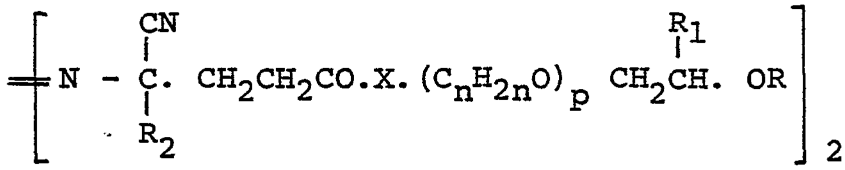 (I)

in which R and $R_1$ are independently hydrogen or a methyl group, $R_2$ is an alkyl group containing from 1 to 4 carbon atoms, X is oxygen or a group $NR_3$ where $R_3$ is hydrogen or a grouping $-(C_nH_{2n}O)_p\ CH_2\overset{R_1}{\overset{|}{C}}H.\ OR$, n has the value 2, 3 or 4 and p is zero or an integer from 1 to 100.

2. Compounds as claimed in claim 1, wherein p is an integer from 1 to 50.

3. Compounds as claimed in claim 1, wherein $R_2$ in formula (I) is a methyl group.

4. A compound as claimed in claim 3, having the formula

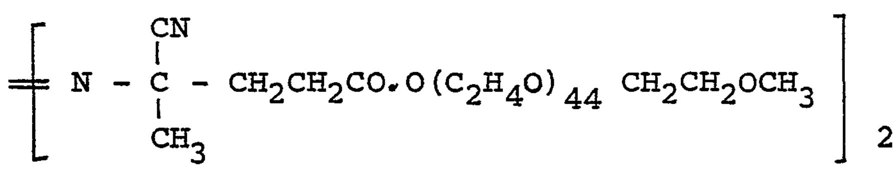

5. A compound as claimed in claim 3, having the formula

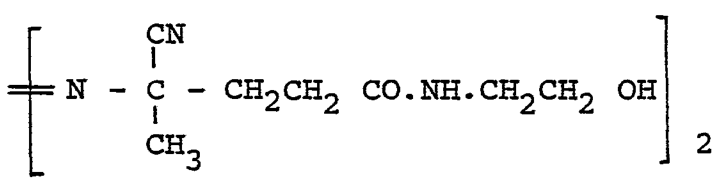

6. A compound as claimed in claim 3, having the formula

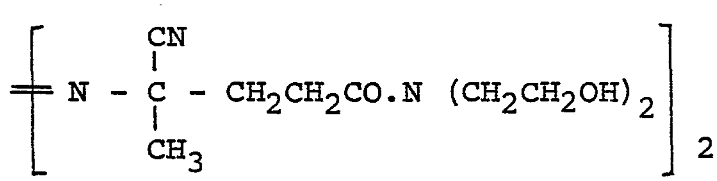

7. A compound as claimed in claim 3, having the formula

$$\left[ = N - \underset{CH_3}{\overset{CN}{\underset{|}{\overset{|}{C}}}} - CH_2CH_2\ CO.NH(C_2H_4O)_{33}\ CH_2CH_2\ OH \right]_2$$

8. A process of free radical-initiated polymerisation of ethylenically unsaturated monomers, wherein the initiator is a compound as claimed in claim 1.

9. A process as claimed in claim 8, which is an aqueous dispersion polymerisation process as described in British Application No. 2 039 497 A and wherein the initiator is preferentially soluble in water.